# EUROPÄISCHE PATENTSCHRIFT

(11) **EP 1 615 891 B1**
(45) Veröffentlichungstag und Bekanntmachung des Hinweises auf die Patenterteilung: **19.06.2013**
(21) Anmeldenummer: 04723902.5
(22) Anmeldetag: 27.03.2004
(51) Int. Cl.: C07D 213/48, C07D 213/56

(54) **VERFAHREN ZUR HERSTELLUNG VON NICOTINALDEHYDEN**
METHOD FOR THE PRODUCTION OF NICOTINALDEHYDES
PROCEDE POUR PRODUIRE DES ALDEHYDES DE NICOTINE

(30) Priorität: 24.04.2003 DE 10318690
(43) Veröffentlichungstag der Anmeldung: 18.01.2006
(73) Patentinhaber: Merck Patent GmbH, 64293 Darmstadt (DE)
(72) Erfinder: BOKEL, Heinz-Hermann, 64287 Darmstadt (DE); BRANDNER, Mike, 64572 Büttelborn (DE); GANTZERT, Ludwig, 64319 Pfungstadt (DE); KNIERIEME, Ralf, 64846 Gross-Zimmern (DE)
(86) Internationale Anmeldenummer: PCT/EP2004/003272
(87) Internationale Veröffentlichungsnummer: WO 2004/094383

(56) Entgegenhaltungen:
- EP-A- 0 580 374
- DOUAT, CELINE ET AL: "Synthesis of N - protected .alpha.-amino aldehydes from their morpholine amide derivatives" TETRAHEDRON LETTERS , 41(1), 37-40 CODEN: TELEAY; ISSN: 0040-4039, 2000, XP002286861
- SAMMAKIA, TAREK ET AL: "Enhanced Selectivities for the Hydroxyl-Directed Methanolysis of Esters Using the 2-Acyl-4-aminopyridine Class of Acyl Transfer Catalysts: Ketones as Binding Sites" JOURNAL OF ORGANIC CHEMISTRY , 65(4), 974-978 CODEN: JOCEAH; ISSN: 0022-3263, 2000, XP002286860

## Beschreibung

Die Erfindung betrifft ein Verfahren zur Herstellung von Nicotinaldehyden durch Reduktion der entsprechenden Nicotinsäure-Morpholinamide.

Nicotinaldehyde sind bedeutende Zwischen- oder Endprodukte in der industriellen Organischen Chemie. Entsprechend substituierte Derivate, wie bspw. Arylnicotinsäurealdehyde stellen unter anderem wertvolle Zwischenprodukte zur Synthese von hochveredelten Endprodukten dar oder sind selbst solche Endprodukte, insbesondere für den Pflanzenschutz, wie z. B. Fungizide, Insektizide, Herbizide oder Pestizide oder zur Herstellung von pharmazeutisch hochwirksamen Substanzen.
Es besteht daher ein Interesse an einer möglichst wirtschaftlichen Produktionsweise dieser Verbindungen im großtechnischen Maßsstab

Als instabile Oxidationsstufe zwischen Alkohol und Carbonsäure sind Aldehyde im Allgemeinen schwer zugänglich. Insbesondere aromatische Aldehyde oxidieren leicht zu den korrespondierenden Carbonsäuren oder sie disproportionieren unter alkalischen Bedingungen zu Alkohol und Carbonsäure. Bei der reduktiven Darstellung von Nicotinaldehyd-Derivaten kommt als zusätzliche Nebenreaktion die Reduktion zum Dihydropyridin hinzu.
Es gibt literaturbekannte Herstellmethoden, die eine selektive Reduktion von Carbonsäurederivaten bis zur Aldehydstufe beschreiben. Diese Methoden setzen im allgemeinen eine Kühlung der Reaktionsmischung voraus, um Über-Reduktionen zu minimieren.
Auch spezielle Methoden zur Reduktion von Nicotinsäurederivaten sind bekannt. So wird beispielsweise in der DE-OS 100 05 150 ein Verfahren zur Herstellung von 5-Arylnicotinaldehyden durch Reduktion der entsprechenden 5-Arylnicotinsäuren mittels katalytischer Hydrierung beschrieben. H.C. Brown und A.Tsukamoto beschrieben in J. Am. Chem. Soc. 81 , S. 502 (1959) die Reduktion von Nicotinsäure-Amiden mit Triethoxy-Lithiumaluminiumhydrid. Dabei wurde jedoch eine niedrige Reaktionstemperatur als unverzichtbar dargestellt und die Ausbeute lag unter 90% d.Theorie.
Weitere bekannte Verfahren zur Herstellung von Nicotinaldehyden durch Reduktion sind in folgender Übersicht dargestellt.

| **Nr.** | **Anzahl Realtions-stufen** | **Derivat der Nicotinsäure** | **Reduktions-mittel** | **Reaktions-beding-ungen** | **Ausbeute** | **Literatur** |
|---|---|---|---|---|---|---|
| 1 | 2 | Diethylamid | Cp₂Zr(H)Cl | Raumtemp. /15 Min. | 99% | J. Am. Chem. Soc. 48 (2000) 11995-11996 |
| 2 | 3 | Nitril | DiBAH (Diisobutylaluminiumhydrid) | Toluol -50°C 2,5 Std. | 96% | J. Org. Chem. 64,26 (1999) 9658-9667 |
| 3 | 3 | Nitril | K-Amyl-(9)-borabicyclononan | THF 25°C | 96% | Tetrahedron Letters 30,28 (1989) 3677-3680 |
| 4 | 3 | Nitril | DiBAH | Toluol -12°C | 70% | J. Med. Chem. 36,8 (1993) 953-966 |
| 5 | 2 | Hydrazid | NaJO₄ | Wasser /NH₃ | 70% | J. Am. Chem. Soc. 74 (1952) 5796 |
| 6 | 3 | N-Methylanilid | LiAIH₄ | THF 0°C | 65% | Angew. Chemie 65 (1953) 525 |
| 7 | 3 | Nitril | DiBAH | THF 0°C | 62% | J. Med. Chem. 35,21 (1992) 3784-3791 |
| 8 | 3 | Sulfonylhydrazid | Na₂CO₃ | 160°C Ethylenglycol | 61% | J. Am. Chem. Soc. 80 (1958) 862 |
| 9 | 3 | Nitril | DiBAH | THF | 61% | J. Med. Chem. 34,9 (1991) 2922-2925 |
| 10 | 2 | primäres Amid | LiAIH(NEt₂)₃ | Raumtemp. 12 Std. | 53% | THL 32,41 (1991) 6903-6904 |
| 11 | 2 | N-Methoxy-N-Methyl-amid | DiBAH | THF -100°C | 51% | Heterocycles 53 (2000) 2183-2190 |

Aus der Übersicht ist zu entnehmen, daß die bekannten Methoden entweder teure Reagenzien benötigen (Beispiele Nr. 1, 3, 10), Rohstoffe verwenden, die nicht in technischen Mengen erhältlich sind (Beispiele Nr. 1, 3, 11), nur mit dem seinerseits dreistufig herzustellenden Nitril durchzuführen sind (Beispiele Nr. 2, 3, 4, 7, 9) oder tiefe Temperaturen erfordern (Beispiele Nr. 2, 4, 11).
Unter Ausbeutegesichtspunkten sind nur die Beispiele Nr. 1, 2 und 3 wirtschaftlich vertretbar. Berücksichtigt man nun noch die Reagenzienkosten, bleibt nur noch das Verfahren nach Beispiel Nr. 2. Letzteres erfordert jedoch ausgehend von der Nicotinsäure drei Reaktionsstufen und ist auf die Einhaltung tiefer Temperaturen angewiesen.

Überraschend wurde nun von den Erfindern der vorliegenden Patentanmeldung gefunden, daß sich Nicotinsäurealdehyde in nahezu quantitativen Ausbeuten durch Reduktion bei Normalbedingungen (Raumtemperatur, drucklos) erhalten lassen, wenn man als Edukte die entsprechenden Morpholinamide einsetzt.
Morpholin-Amide von Nicotinsäure und deren Derivaten waren bisher noch nicht als Aldehyd-Vorstufen bekannt.

Gegenstand der vorliegenden Erfindung ist somit ein Verfahren zur Herstellung von Nicotinaldehyden dadurch gekennzeichnet, daß
als Edukte der Reduktion die entsprechenden Morpholinamide eingesetzt werden. Vorzugsweise wird das besagte Verfahren bei Raumtemperatur und drucklos (unter Atmosphärendruck) durchgeführt.

Erfindungsgemäß wird als Reduktionsmittel dabei AIH(OEt)₃ eingesetzt.

In einer bevorzugten Ausführungsform werden als Edukte Nicotinsäure-Morpholinamide der Formel I 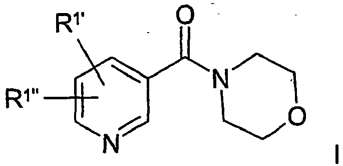 worin
- R¹', R¹": jeweils unabhängig voneinander H, Hal, A, OA, CH₂R² oder Ar,
- R²: OA oder NA₂,
- A: unverzweigtes oder verzweigtes Alkyl mit 1-10 C-Atomen, worin eine oder zwei CH₂-Gruppen durch O- oder S-Atome und/oder durch -CH=CH-Gruppen und/oder auch 1-7 H-Atome durch F ersetzt sein können,
- Ar: ungesättigtes, teilweise oder ganz gesättigtes, unsubstituiertes oder ein- oder mehrfach durch Hal, A, OA, NA₂, NO₂, NASO₂A, SO₂NA SO₂A substituiertes ein- oder mehrkerniges homo- oder heterocyclisches System mit den Heteroatomen O, N, S und
- Hal: F, Cl, Br oder I
bedeutet,
zu Nicotinaldehyden der Formel II 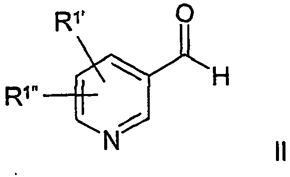 reduziert.

Dabei haben die vorgenannten Reste vozugsweise folgende Bedeutungen:
R^{1'}, R^{1"} bedeutet jeweils unabhängig voneinander H, Hal, A, OA, CH₂R² oder Ar, wobei A, Ar, Hal und R² eine der nachfolgend beschriebenen Bedeutungen haben. R^{1'} R^{1"} sind insbesondere Wasserstoff, Methoxy, Ethoxy, Propoxy, Butoxy, Fluor, Chlor, Brom, lod, Phenyl oder in o,m oder p-Stellung substituiertes Phenyl. Besonders bevorzugt ist R^{1'} p-Fluorphenyl oder Brom und R^{1"} gleichzeitig Wasserstoff.

Hal bedeutet Fluor, Chlor, Brom oder lod, bevorzugt Fluor, Chlor oder Brom.

R² bedeutet OA oder NA₂, wobei A die vor- und nachstehend genannte Bedeutung hat.

A bedeutet Alkyl, ist unverzweigt (linear) oder verzweigt, und hat 1, 2, 3, 4, 5, 6, 7, 8, 9 oder 10 C-Atome.
A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1-oder 2-Ethylbutyl, 1-Ethyl-1-methylpropyl, 1-Ethyl-2-methylpropyl, 1,1,2-oder 1,2,2-Trimethylpropyl, weiter bevorzugt z.B. Trifluormethyl.
A bedeutet ganz besonders bevorzugt Alkyl mit 1-6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl. Ferner bedeutet A Cycloalkyl, vorzugsweise Cyclopropyl, Cyclobutyl, Cyclopentyl, Cyclohexyl, Cycloheptyl, Cyclooctyl oder 2,6,6-Trimethyl-bicyclo[3.1.1]heptyl, jedoch ebenfalls mono- oder bicyclische Terpene, vorzugsweise p-Menthan, Menthol, Pinan, Bornan oder Campher, wobei jede bekannte stereoisomere Form eingeschlossen ist, oder Adamantyl. Für Campher bedeutet dies sowohl L-Campher als auch D-Campher.

Ar bedeutet ein ungesättigtes, teilweise oder ganz gesättigtes, unsubstituiertes oder ein- oder mehrfach durch Hal, A, OA, NA₂, NO₂, NASO₂A, SO₂NA₂, SO₂A substituiertes ein- oder mehrkerniges homo- oder heterocyclisches System mit den Heteroatomen O, N, S.
Bevorzugte cyclische Systeme sind unsubstituiertes oder substituiertes Phenyl, Naphthyl oder Biphenyl, im einzelnen bevorzugt Phenyl, o-, m- oder p-Tolyl, o-, m- oder p-Cyanphenyl, o-, m- oder p-Methoxyphenyl, o-, m-oder p-Fluorphenyl, o-, m- oder p-Bromphenyl, o-, m- oder p- Chlorphenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2-Fluor-4-Bromphenyl, 2,5-Difluor-4-bromphenyl.

Besonders bevorzugte Edukte für die erfindungsgemäße Aldehydsynthese sind 5-(4-Fluorphenyl)-Nicotinsäure-Morpholinamid und 5-Bromnicotinsäure-Morpholinamid.

Gegenstand der vorliegenden Erfindung ist dementsprechend auch die Verwendung von Nicotinsäure-Morpholinamiden, vorzugsweise 5-(4-Fluorphenyl)-Nicotinsäure-Morpholinamid oder 5-Bromnicotinsäure-Morpholinamid, zur Herstellung der entsprechenden NicotinsäureAldehyde.

Gegenstand der vorliegenden Erfindung sind ferner 5-(4-Fluorphenyl)-Nicotinsäure-Morpholinamid und 5-Bromnicotinsäure-Morpholinamid als Edukte der erfindungsgemäßen Synthese.

Die erfindungsgemäße Umsetzung erfolgt in der Regel in einem inerten Lösungsmittel. Als inerte Lösungsmittel für die zuvor beschriebenen Umsetzungen eignen sich z.B. Kohlenwasserstoffe wie Hexan, Petrolether, Benzol, Toluol oder Xylol; Ether wie Diethylether, Diisopropylether, Tetrahydrofuran (THF) oder Dioxan; Glykolether wie Ethylenglykoldimethylether (Diglyme); oder Gemische der genannten Lösungsmittel. Besonders bevorzugt sind Ether, insbesondere Tetrahydrofuran.
Die Menge des Lösungsmittels ist nicht kritisch, in der Regel können 5 g bis 500 g, vorzugsweise 10 g bis 100 g Lösungsmittel je g Edukt zugesetzt werden.

Die Reaktionstemperatur für die zuvor beschriebenen Umsetzungen liegt je nach den angewendeten Bedingungen zwischen etwa -10° und 200°, normalerweise zwischen -10° und 100°, insbesondere zwischen 0° und 50°, bevorzugt jedoch bei 10° bis 40°, besonders bevorzugt bei Raumtemperatur.
Die Reaktionszeit liegt je nach den angewendeten Bedingungen zwischen einigen Sekunden und mehreren Stunden, vorzugsweise zwischen 1 Minute und 3 Stunden. In aller Regel wird die erfindungsgemäße Umsetzung jedoch nach 0,1 bis 1,5 Stunden beendet sein.

Unter den "angewendeten Bedingungen" im Sinne dieser Erfindung werden das Substitutionsmuster des Nicotinsäure-Morpholinamids, die Art und Menge des Lösemittels, die Art und Menge des Reduktionsmittels, die Reaktionsdauer, die Reaktionstemperatur sowie weitere Details der Reaktionsführung wie bspw. die Rührergeschwindgkeit oder die sonstige Beschaffenheit des Reaktionsgefäßes verstanden.

In der Regel wird das Ende der erfindungsgemäßen Reduktion zum Aldehyd durch geeignete Analysemethoden, z. B. Dünnschichtchromatographie oder HPLC, ermittelt und die Reduktion unterbrochen.
Durch übliche Aufarbeitungschritte wie z. B. Wasser- oder Säurezugabe zum Realctionsgemisch und Extraktion können die erfindungsgemäßen Nicotinsäurealdehyde nach Entfernung des Lösungsmittels erhalten werden. Es kann vorteilhaft sein, zur weiteren Reinigung des Produktes eine Destillation oder Kristallisation anzuschließen.

Die als Ausgangsstoffe für das erfindungsgemäße Verfahren verwendeten Nicotinsäure-Morpholinamide können nach an sich bekannten Methoden hergestellt werden, wie sie in der Literatur beschrieben sind (z.B. in Standardwerken wie Houben-Weyl, Methoden der organischen Chemie, Georg-Thieme-Verlag, Stuttgart) und zwar unter Reaktionsbedingungen, die für die genannten Umsetzungen bekannt und geeignet sind. Man kann aber auch von an sich bekannten, hier nicht näher erwähnten Varianten Gebrauch machen.

Im allgemeinen wird man dabei wie folgt vorgehen:
Die Nicotinsäure wird mit einem geeigneten Ragenz wie z.B. Thionylchlorid in das Säurechlorid überführt, das anschliessend mit dem gewünschten Amin zum Amid umgesetzt wird.

Zum Schutz von Substituenten vor unerwünschten Reaktionen bei der erfindungsgemäßen Reduktion und/oder sich daran anschließenden Aufarbeitungsschritten setzt man gegebenfalls Schutzgruppen ein, die nach erfolgter Reduktion des Nicotinsäure-Morpholinamid wieder abgespalten werden. Methoden zur Verwendung von Schutzgruppen sind beispielsweise in Theodora W. Green, Peter G. M. Wuts: Protective Groups in Organic Synthesis, 3rd Edition John Wiley & Sons (1999) beschrieben.

Auch ohne weitere Ausführungsformen wird davon ausgegangen, daß ein Fachmann die obige Beschreibung im weitesten Umfang nutzen kann. Die bevorzugten Ausführungsformen sind deswegen lediglich als beschreibende, keineswegs als in irgendeiner Weise limitierende Offenbarung aufzufassen.

### Beispiel 1: Herstellung von 5-(4-Fluorphenyl)-Nicotinsäurealdehyd aus 5-(4-Fluorphenyl)-pyridin-3-carbonsäure-Morpholinamid

### (a) Synthese der Vorstufe 5-(4-Fluorphenyl)-pyridin-3-carbonsäure

Zunächst wird 5-(4-Fluorphenyl)-pyridin-3-carbonsäure durch Suzuki-Kupplung (N. Miyaura, A. Suzuki, Chem. Rev. 95, 2457 (1995)) hergestellt, indem man 5-Brom-nicotinsäure mit p-Fluorbenzolboronsäure (beides kommerziell erhältlich) unter an sich bekannten Reaktionsbedingungen zu 5-(4-Fluorphenyl)-pyridin-3-carbonsäure umgesetzt.

### (b1) Synthese des 5-(4-Fluorphenyl)-pyridin-3-carbonsäure-Morpholinamids - Variante 1

25,6 g 5-(4-Fluorphenyl)nicotinsäure werden in 200 ml Toluol vorgelegt und dann bei Raumtemperatur mit 25,1 g Thionylchlorid versetzt. Nun wird 18 Stunden auf 90°C erwärmt und anschliessend das nicht umgesetzte Thionylchlorid und ein Teil des Lösemittels abdestilliert. Nach Auffüllen des abdestillierten Volumens mit Toluol werden bei 80 bis 100°C 12,4 g Morpholin zugegeben und die Reaktionsmischung nach 2 Stunden abgekühlt. Durch Zugabe von Natronlauge wird pH 8 eingestellt und das Produkt durch Extraktion mit Toluol abgetrennt. Nach Entfärbung mit Aktivkohle und Abdestillieren des Lösemittels bleiben 27,7 g 5-(4-Fluorphenyl)-pyridin-3-carbonsäure-Morpholinamid als Feststoff zurück (Schmelzpunkt: 100-102°C, Ausbeute: 81% d.Th.)

### (b2) Synthese des 5-(4-Fluoroheny))-pyridin-3-carbonsäure-Morpholinamids - Variante 2

Zu einer Lösung von 14,3 g 5-Bromnicotinsäure-Morpholinamid in 100 g THF werden 1,2 g Pd[P(Ph)₃]₄ und 7,6 g p-Fluorbenzolboronsäure gegeben. Anschliessend wird bei 65 °C eine Lösung aus 8,0 g Na₂CO₃ in 25 g Wasser unter Rühren zugetropft. Nach 16 Stunden wird die Realctionsmischung abgekühlt und am Rotationsverdampfer eingeengt. Der Rückstand wird in Dichlormethan aufgenommen, mit Aktivkohle versetzt und filtriert. Nach mehrfacher Extraktion des Filtrats mit Wasser und Einengen am Rotationsverdampfer erhält man einen Rückstand von 15,7 g, der laut HPLC 89% 5-(4-Fluorphenyl)-pyridin-3-carbonsäure-Morpholinamid enthält (Netto-Ausbeute 93% d.Th.). Nach Umkristallisieren aus Ethylacetat erhält man daraus 8,0 g 5-(4-Fluorphenyl)-pyridin-3-carbonsäure-Morpholinamid mit einer HPLC-Reinheit von 99,6% (53,3 %d.Th.).

### (c) Herstellung von 5-(4-Fluorphenyl)-Nicotinsäurealdehyd

Es werden 6,0 g 5-(4-Fluorphenyl)-pyridin-3-carbonsäure-morpholinamid aus Beispiel 1 (b) in 30 ml THF gelöst und bei 30°C bis 35°C innerhalb von 10 Minuten mit 57 g einer 13,6%igen LiAIH(OEt)₃-Lösung in THF versetzt. Nach 1 Stunde werden 30 ml 12,5%ige Schwefelsäure zugegeben und die organische Phase wird abgetrennt. Die wässrige Phase wird mit Schwefelsäure auf pH 1 eingestellt und mehrfach mit Methyl-tert.-Butyl- Ether extrahiert. Die organischen Phasen werden nun vereinigt, einmal mit Wasser extrahiert und danach eingeengt. Es bleibt ein Rückstand von 4,3 g mit einem Gehalt von 97 Gew.% 5-(4-Fluorphenyl)-pyridin-3-carbaldehyd zurück (Ausbeute = 98% d.Theorie).

### Beispiel 2 (Vergleichsbeispiel zu Beispiel 1, Verwendung des Piperidin- anstelle des Morpholinamids): Herstellung von 5-(4-Fluorphenyl)-Nicotinsäurealdehyd aus 5-(4-Fluorphenyl)-pyridin-3-carbonsäure-Piperidinamid

36,7 g 10%ige LiAIH₄-Lösung werden mit 75 g THF verdünnt und dann bei 0 °C mit einer Mischung aus 8,88 g Ethylacetat und 75 g THF versetzt. Bei -7°C wird eine Lösung aus 6,8 g 5-(4-Fluorphenyl)-pyridin-3-carbonsäure-piperidinamid in 24,7 ml THF zugegeben. Nach drei Stunden wird die Mischung 190 g 10%ige Schwefelsäure gegeben. Mit Natronlauge wird auf pH 3 eingestellt und das THF danach weitgehend abdestilliert. Nach Extraktion mit Methyl-tert.-Butyl-Ether und Einengen bleiben 2,6 g Feststoff zurück (Gehalt laut HPLC 67 Flächen-%, entspr. 36% d.Theorie)

### Beispiel 3: Herstellung von 5-Bromnicotinaldehyd aus 5-Brom-pyridin-3-carbonsäure-Morpholinamid (Vergleichsbeispiel)

### (a) Synthese des 5-Brom-pyridin-3-carbonsäure-Morpholinamids

50,4 g 5-Bromnicotinsäure und 87,5 g Morpholin werden in 200 ml Xylol zum Rückfluss erhitzt und das entstandene Wasser abdestilliert. Nach Abkühlen der Reaktionsmischung wird dreimal mit 10%iger Natronlauge und dann zweimal mit Wasser extrahiert. Nach Abdestillieren des Xylols wird der Rückstand aus Ethylacetat umkristallisiert. Die Ausbeute nach Trocknen beträgt 19,2 g (28,3% d.Th.). Schmelzpunkt 80 °C.

### (b) Herstellung von 5-Brom-Nicotinsäurealdehyd

1,75 g Lithiumaluminiumhydrid-Pulver werden in 64 g THF suspendiert. Anschliessend wird unter Kühlung eine Mischung aus 5,9 g Ethylacetat und 28 g THF zugetropft. Nach 30 Minuten wird diese Reaktionsmischung bei 0°C bis 10°C zu einer Lösung von 5,0 g 5-Brom-pyridin-3-carbonsäure-morpholinamid aus Beispiel 3(a) in 30 g THF getropft (dies entspricht 150% Überschuss des Reduktionsmittels). Nach 1 Stunde wird die Reaktionsmischung auf 35ml 12%ige Schwefelsäure gegossen und die organische Phase zum Rückstand eingeengt. Nach Umkristallisation aus MTB-Ether und Trocknen erhält man 1,91 g Produkt (= 55,7% d.Th). Schmelzpunkt 95 °C.

Wie aus dem Vergleich der jeweiligen Reaktionsausbeuten der Beispiele 1 und 2 ersichtlich ist, führt die Verwendung des Nicotinsäure-Piperidinamids, eines alternativen - zu dem Morpholinamid strukturell jedoch eng verwandten - Nicotinsäureamids zu einer deutlich schlechteren Umsetzung.

Dagegen ist bei Verwendung des Morpholinamids sogar noch mit einem grossen Überschuss des Reduktionsmittels (Beispiel 3) eine bessere Ausbeute zu erzielen als bei korrekter Stöchiometrie in Beispiel 2, bei dem Piperidin als Aminkomponente verwendet wird.

## Patentansprüche

1. Verfahren zur reduktiven Herstellung von Nicotinaldehyden, **dadurch gekennzeichnet, dass** als Edukte der Reduktion die entsprechenden Nicotinsäure-Morpholinamide eingesetzt werden, und dass als Reduktionsmittel LiAIH(OEt)₃ eingesetzt wird.

2. Verfahren nach Anspruch 1, **dadurch gekennzeichnet, dass** als Edukte Nicotinsäure-Morpholinamide der Formel 1 eingesetzt werden 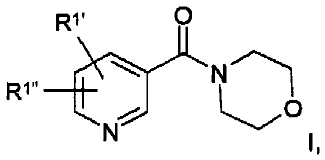 worin
R¹', R¹" jeweils unabhängig voneinander H, Hal, A, OA, CH₂R² oder Ar,
R² OA oder NA₂,
A unverzweigtes oder verzweigtes Alkyl mit 1-10 C-Atomen, worin eine oder zwei CH₂-Gruppen durch O- oder S-Atome und/oder durch -CH=CH-Gruppen und/oder auch 1-7 H-Atome durch F ersetzt sein können,
Ar ungesättigtes, teilweise oder ganz gesättigtes, unsubstituiertes oder ein- oder mehrfach durch Hal, A, OA, NA₂, NO₂, NASO₂A, SO₂NA, SO₂A substituiertes ein- oder mehrkerniges homo- oder heterocyclisches System mit den Heteroatomen O N, S, und
Hal F, Cl, Br oder I bedeutet.

3. Verfahren nach Anspruch 1 oder 2, **dadurch gekennzeichnet, dass** als Edukt 5-(4-Fluorphenyl)-Nicotinsäure-Morpholinamid eingesetzt wird.

4. Verfahren nach Anspruch 1 oder 2, **dadurch gekennzeichnet, dass** als Edukt 5-Brom-pyridin-3-carbonsäure-Morpholinamid eingesetzt wird.

5. Verwendung von Nicotinsäure-Morpholinamiden zur reduktiven Herstellung der entsprechenden Nicotinaldehyde, **dadurch gekennzeichnet, dass** als Reduktionsmittel LiAIH(OEt)₃ eingesetzt wird..

6. Verwendung nach Anspruch 5, wobei die Nicotinsäure-Morpholinamide der Formel gemäß Anspruch 2 entsprechen und die Reste R¹' und R¹" die in Anspruch 2 angegebene Bedeutung haben.

7. Edukte der Formel I gemäß Anspruch 2, ausgewählt aus einer Gruppe bestehend aus
(a) 5-(4-Fluorphenyl)-Nicotinsäure-Morpholinamid,
(b) 5-Bromnicotinsäure-Morpholinamid.

## Claims

1. Process for the reductive preparation of nicotinaldehydes, **characterised in that** the starting materials employed for the reduction are the corresponding nicotinic acid morpholinamides, and **in that** the reducing agent employed is LiAIH(OEt)₃.

2. Process according to Claim 1, **characterised in that** the starting materials employed are nicotinic acid morpholinamides of the formula I 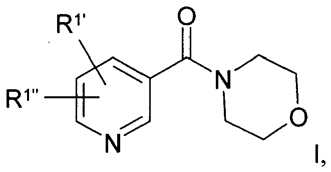 in which
R¹' R¹" each, independently of one another, denote H, Hal, A, OA, CH₂R² or Ar,
R² denotes OA or NA₂,
A denotes unbranched or branched alkyl having 1-10 C atoms, in which one or two CH₂ groups may be replaced by O or S atoms and/or by -CH=CH- groups and/or in addition 1-7 H atoms may be replaced by F,
Ar denotes an unsaturated, partially or fully saturated, mono- or polycyclic homo- or heterocyclic system containing the heteroatoms O, N, S which is unsubstituted or mono- or polysubstituted by Hal, A, OA, NA₂, NO₂, NASO₂A, SO₂NA, SO₂A,
and
Hal denotes F, Cl, Br or I.

3. Process according to Claim 1 or 2, **characterised in that** the starting material employed is 5-(4-fluorophenyl)nicotinic acid morpholinamide.

4. Process according to Claim 1 or 2, **characterised in that** the starting material employed is 5-bromopyridine-3-carboxylic acid morpholin-amide.

5. Use of nicotinic acid morpholinamides for the reductive preparation of the corresponding nicotinaldehydes, **characterised in that** the reducing agent employed is LiAIH(OEt)₃.

6. Use according to Claim 5, where the nicotinic acid morpholinamides conform to the formula I according to Claim 2 and the radicals R¹' and R¹" have the meaning indicated in Claim 2.

7. Starting materials of the formula I according to Claim 2, selected from a group consisting of
(a) 5-(4-fluorophenyl)nicotinic acid morpholinamide,
(b) 5-bromonicotinic acid morpholinamide.

## Revendications

1. Procédé de préparation réductrice de nicotinaldéhydes, **caractérisé en ce que** les produits de départ employés pour la réduction sont les morpholinamides d'acide nicotinique correspondants, et **en ce que** l'agent réducteur employé est LiAIH(OEt)₃.

2. Procédé selon la revendication 1, **caractérisé en ce que** les produits de départ employés sont les morpholinamides d'acide nicotinique de formule I 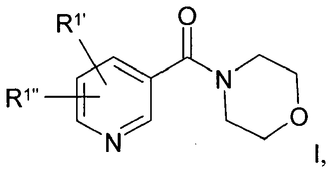 dans laquelle
R¹', R¹" désignent chacun, indépendamment l'un de l'autre, H, Hal, A, OA, CH₂R² ou Ar,
R² désigne OA ou NA₂,
A désigne alkyle non ramifié ou ramifié ayant 1-10 atomes de C, où un ou deux groupements CH₂ peuvent être remplacés par des atomes de O ou S et/ou par des groupements -CH=CH-et/ou en outre 1-7 atomes de H peuvent être remplacés par F,
Ar désigne un système mono- ou polycyclique homo- ou hétérocyclique, non saturé, partiellement ou totalement saturé, contenant les hétéroatomes O, N, S qui est non substitué ou mono- ou polysubstitué par Hal, A, OA, NA₂, NO₂, NASO₂A, SO₂NA, SO₂A,
et
Hal désigne F, CI, Br ou I.

3. Procédé selon la revendication 1 ou 2, **caractérisé en ce que** le produit de départ employé est le morpholinamide de l'acide 5-(4-fluorophényl)-nicotinique.

4. Procédé selon la revendication 1 ou 2, **caractérisé en ce que** le produit de départ employé est le morpholinamide de l'acide 5-bromopyridine-3-carboxylique.

5. Utilisation de morpholinamides d'acide nicotinique pour la préparation réductrice des nicotinaldéhydes correspondants, **caractérisée en ce que** l'agent réducteur employé est LiAIH(OEt)₃.

6. Utilisation selon la revendication 5, dans laquelle les morpholinamides d'acide nicotinique répondent à la formule I selon la revendication 2 et les radicaux R¹' et R¹" revêtent la signification indiquée selon la revendication 2.

7. Produits de départ de formule I selon la revendication 2, choisis dans le groupe constitué par :
(a) le morpholinamide d'acide 5-(4-fluorophényl)nicotinique,
(b) le morpholinamide d'acide 5-bromonicotinique.
